# EUROPÄISCHE PATENTSCHRIFT

(11) **EP 1 642 553 B1**
(45) Veröffentlichungstag und Bekanntmachung des Hinweises auf die Patenterteilung: **02.10.2013**
(21) Anmeldenummer: 05017037.2
(22) Anmeldetag: 05.08.2005
(51) Int. Cl.: A61F 2/44

(54) **Zwischenwirbelprothese**
Intervertebral prosthesis
Prothèse intervertébrale

(30) Priorität: 30.09.2004 US 955103
(43) Veröffentlichungstag der Anmeldung: 05.04.2006
(73) Patentinhaber: Weber, Helmut, 78576 Emmingen-Liptingen (DE)
(72) Erfinder: Weber, Helmut, 78576 Emmingen-Liptingen (DE)
(74) Vertreter: Patentanwälte Westphal, Mussgnug & Partner

(56) Entgegenhaltungen:
- WO-A-02/089701
- DE-U1- 20 315 611
- DE-U1-202004 009 542
- US-A1- 2004 002 761
- US-B1- 6 368 350

## Beschreibung

Die Erfindung betrifft eine Zwischenwirbelprothese gemäß dem Oberbegriff des Patentanspruchs 1.

Bekannt sind beispielsweise aus der EP 0 176 728 A1 oder der WO 02/080818 A1 dreiteilige Zwischenwirbelprothesen bestehend aus zwei Prothesenplatten, zwischen welchen ein Prothesenkern angeordnet ist. Die Prothesenplatten bestehen aus einer kreisförmigen oder ellipsenförmigen Scheibe, welche auf einer Seite eine kugelschalenförmige Vertiefung aufweisen. Der Prothesenkern besteht aus einer kreisförmigen Scheibe, welche beidseitig jeweils ein axial angeordnetes Kugelsegment und einen am äußeren Rand umlaufenden axial vorspringenden Ringsteg aufweist. Im zusammengesetzten Zustand der Zwischenwirbelprothese liegen die Kugelsegmente des Prothesenkerns in den kugelschalenförmigen Vertiefungen der beiden Prothesenplatten.

Wird die Zwischenwirbelprothese als Ersatz für eine krankhafte Bandscheibe, welche operativ entfernt wurde, in den Zwischenwirbelraum eingebracht, werden die beiden Prothesenplatten jeweils an einem Wirbel befestigt und der Prothesenkern zwischen die Prothesenplatten eingesetzt. Das Zusammenspiel der Kugelsegmente mit den kugelschalenförmigen Vertiefungen ermöglicht eine Drehbewegung der beiden benachbarten Wirbelkörper.

Bekannt sind auch beispielsweise aus der WO 02/080818 A1 dreiteilige Zwischenwirbelprothesen mit einem Prothesenkern, welcher nur ein Kugelsegment und anstelle des zweiten Kugelsegments einen flachen Abschnitt aufweist. Dieser flache Abschnitt ist in einer entsprechend ausgeformten Vertiefung der einen Prothesenplatte fixiert, da die Bewegung eines Kugelsegments in einer kugelschalenförmigen Vertiefung für eine Kipp- und Drehbewegung der beiden Prothesenplatten gegeneinander ausreichend ist.

Die beschriebenen Zwischenwirbelprothesen ermöglichen eine Drehbewegung der beiden mit den Prothesenplatten verbundenen Wirbelkörper um einen Drehpunkt, welcher auf der Achse des rotationssymmetrischen Prothesenkerns liegt. Bei einer Drehung und Bewegung eines Oberkörpers mit gesunder Wirbelsäule liegt jedoch der Drehpunkt in der Regel nicht im Zentrum der Bandscheibe, sondern außerhalb des Zwischenwirbelraumes. Die Zwischenwirbelprothesen gemäß dem Stand der Technik weisen daher den Nachteil auf, die natürliche Bewegung der Wirbelsäule nicht optimal zu simulieren.

Die WO 02/089701 A2 beschreibt eine Zwischenwirbelprothese gemäß dem Oberbegriff des Anspruchs 1. Als weiterer Stand der Technik werden die Dokumente US 2004/0002761 A1, DE 203 15 611 U1 und DE 20 2004 009 542 U1 genannt.

Die Aufgabe der Erfindung besteht daher darin, einen Zwischenwirbelprothese bereitzustellen, welche ein verbessertes Simulationsverhalten der natürlichen Bewegung der Wirbelsäule aufweist.

Die Erfindung wird gelöst durch eine Zwischenwirbelprothese mit den kennzeichnenden Merkmalen des Patentanspruchs 1.

Vorteilhafte Ausgestaltungen und Weiterbildungen der Erfindung sind in den Unteransprüchen angegeben.

Erfindungsgemäß weist die Zwischenwirbelprothese einen Prothesenkern auf, welcher ein Kugelsegment und einen abgeflachten Abschnitt aufweist, wobei der flache Abschnitt des Prothesenkerns in eine im Wesentlichen ebene Vertiefung einer der beiden Prothesenplatten eingreift, wobei erfindungsgemäß der flache Abschnitt des Prothesenkerns in der ebenen Vertiefung verschiebbar bewegbar ist. Durch eine derartige Ausgestaltung der Zwischenwirbelprothese wird zusätzlich zu den möglichen Kippbewegungen der beiden Prothesenplatten gegeneinander eine Relativbewegung der beiden Prothesenplatten in einer Ebene parallel zur Ebene der Prothesenplatten gegeneinander ermöglicht. Bei einer Bewegung der Wirbelsäule liegt somit der Drehpunkt der Bewegung nicht notwendigerweise auf der Achse des Prothesenkerns. Durch die Kombination von Kipp- und Translationsbewegung kann ein Drehpunkt außerhalb des Zwischenwirbelbereichs simuliert werden.

Weiterhin weist die im Wesentlichen ebene Vertiefung einen im Wesentlichen runden Querschnitt auf. Derartige Vertiefungen sind besonders einfach fertigungstechnisch herzustellen.

Der flache Abschnitt des Prothesenkerns weist einen im Wesentlichen runden Querschnitt auf. Eine derartige Ausgestaltung des Prothesenkerns ist ebenfalls besonders einfach zu fertigen. Insbesondere ermöglicht das Zusammenspiel einer im Wesentlichen runden Vertiefung und eines im Wesentlichen runden flachen Abschnitts des Prothesenkerns eine optimale Bewegung des Prothesenkerns in Bezug auf die Prothesenplatte, da keinerlei Verkantungen des Prothesenkerns in der ebenen Vertiefung der Prothesenplatte auftreten können und sich der Prothesenkern beliebig drehen kann.

Dabei ist der Durchmesser des flachen Abschnitts kleiner als der Durchmesser der im Wesentlichen ebenen Vertiefung. Auf diese Weise wird die Bewegung des flachen Abschnitts des Prothesenkerns in der im Wesentlichen ebenen Vertiefung ermöglicht.

Erfindungsgemäß weitet sich der flache Abschnitt des Prothesenkerns ausgehend von der ebenen Oberfläche des Kugelsegments konusförmig nach außen auf. Durch eine derartige Ausgestaltung des flachen Abschnitts des Prothesenkerns wird ein Hintergreifen eines an der Prothesenplatte ausgebildeten Vorsprungs ermöglicht, so dass ein Herausdrücken des Prothesenkerns aus dem Zwischenraum zwischen den beiden Prothesenplatten zuverlässig verhindert wird.

Die im Wesentlichen ebene Vertiefung verjüngt sich ausgehend von der Grundfläche konusförmig nach innen. Dadurch wird ein umlaufender Vorsprung gebildet, welche in eine entsprechend ausgebildete umlaufende Nut des Prothesenkerns eingreift und ein Herausdrücken des Prothesenkerns aus dem Zwischenraum zwischen den beiden Prothesenplatten zuverlässig verhindert.

Bei einer vorteilhaften Weiterbildung der Erfindung weist die Grundfläche der im Wesentlichen ebenen Vertiefung eine Wölbung auf. Eine derartige Wölbung ermöglicht zusätzlich zu der Translationsbewegung des Prothesenkerns in der im Wesentlichen ebenen Vertiefung der Prothesenplatte eine geringe Kippbewegung, welche das Simulationsverhalten der Zwischenwirbelprothese weiter verbessert.

Vorzugsweise ist der Radius der Wölbung der im Wesentlichen ebenen Vertiefung fünf bis zehnmal so groß wie der Radius der kugelschalenförmigen Vertiefung. Die Grundfläche der im Wesentlichen ebenen Vertiefung ist somit weiterhin im Wesentlichen eben und ermöglicht nur sehr geringe Kippbewegungen, während der überwiegende Teil der Kippbewegung durch das Zusammenspiel des Kugelsegments des Prothesenkerns in der kugelschalenförmigen Vertiefung der Prothesenplatte bewirkt wird.

Vorteilhafterweise ist die Grundfläche des flachen Abschnitts gewölbt. Insbesondere bei einer Abstimmung der Wölbung des flachen Abschnitts auf die Wölbung der Grundfläche der ebenen Vertiefung wird eine Translationsbewegung des Prothesenkerns in der im Wesentlichen ebenen Vertiefung ermöglicht, welche zugleich eine geringfügige Kippbewegung zulässt, wobei jedoch weiterhin der Prothesenkern mit der gesamten Grundfläche des im Wesentlichen flachen Abschnitts auf der Grundfläche der im Wesentlichen ebenen Vertiefung aufliegt.

Vorzugsweise entspricht der Winkel zwischen der Außenfläche des flachen Abschnitts und der Grundfläche des flachen Abschnitts des Prothesenkerns dem Winkel der Außenfläche der im Wesentlichen ebenen Vertiefung gegenüber der Grundfläche der im Wesentlichen ebenen Vertiefung der Prothesenplatte. Auf diese Weise wird eine Führung des Prothesenkerns in der Vertiefung der Prothesenplatte nach Art einer Schwalbenschwanzführung ermöglicht. Der Prothesenkern wird somit besonders zuverlässig im Zwischenraum in der im Wesentlichen ebenen Vertiefung der Prothesenplatte und somit zwischen den beiden Prothesenplatten gehalten.

Vorzugsweise sind die Oberflächen der beiden Prothesenplatten ausgehend von dem Außenrand der kugelschalenförmigen Vertiefung bzw. der im Wesentlichen ebenen Vertiefung mit einer Neigung nach außen abfallend ausgebildet. Bei einem Verkippen der beiden Prothesenplatten gegeneinander kann somit bei entsprechender Ausbildung der Neigung erreicht werden, dass bei einer Maximalverkippung der beiden Prothesenplatten gegeneinander die Außenbereiche der beiden Prothesenplatten entlang einer Geraden anstelle nur eines Auflagepunktes auf den Innenflächen aufliegen, wodurch die Belastung besser verteilt und Beschädigung der Protheseplatten vermieden werden.

Ein Ausführungsbeispiel der Erfindung wird anhand der folgenden Figuren ausführlich erläutert.

Es zeigt
- Figur 1: eine Seitenansicht eines Prothesenkerns einer Zwischenwirbelprothese gemäß der Erfindung,
- Figur 2a: eine Draufsicht auf die Innenfläche der zweiten Prothesenplatte einer Zwischenwirbelprothese gemäß der Erfindung,
- Figur 2b: eine Draufsicht auf die Außenfläche der Prothesenplatte aus Figur 2a,
- Figur 3a: eine Seitenansicht eines Ausführungsbeispiels einer Zwischenwirbelprothese gemäß der Erfindung,
- Figur 3b: eine weitere Seitenansicht der Zwischenwirbelprothese aus Figur 3a,
- Figur 4a: einen Axialschnitt entlang der Nebenachsen der beiden Prothesenplatten durch die erfindungsgemäße Zwischenwirbelprothese mit gegeneinander verkippten Prothesenplatten,
- Figur 4b: einen Axialschnitt entlang der Hauptachsen der beiden Prothesenplatten durch die erfindungsgemäße Zwischenwirbelprothese aus Figur 4a mit gegeneinander verkippten Prothesenplatten,
- Figur 5a: einen Axialschnitt entlang der Nebenachsen der beiden Prothesenplatten durch die erfindungsgemäße Zwischenwirbelprothese mit gegeneinander verkippten Prothesenplatte in einer weiteren Position,
- Figur 5b: einen Axialschnitt entlang der Hauptachsen der beiden Prothesenplatten durch die erfindungsgemäße Zwischenwirbelprothese aus Figur 5a mit gegeneinander verkippten Prothesenplatten in einer weiteren Position,
- Figur 6a: einen Axialschnitt entlang der Nebenachsen der beiden Prothesenplatten durch die erfindungsgemäße Zwischenwirbelprothese mit zueinander parallel ausgerichteten Prothesenplatten,
- Figur 6b: einen Axialschnitt entlang der Hauptachsen der beiden Prothesenplatten durch die erfindungsgemäße Zwischenwirbelprothese aus Figur 6a mit zueinander parallel ausgerichteten Prothesenplatten,
- Figur 7a: einen Axialschnitt entlang der Nebenachsen der beiden Prothesenplatten durch die erfindungsgemäße Zwischenwirbelprothese mit gegeneinander verkippten Prothesenplatten in einer weiteren Position,
- Figur 7b: einen Axialschnitt entlang der Hauptachsen der beiden Prothesenplatten durch die erfindungsgemäße Zwischenwirbelprothese aus Figur 7a mit gegeneinander verkippten Prothesenplatten in einer weiteren Position,
- Figur 8a: einen Axialschnitt entlang der Nebenachsen der beiden Prothesenplatten durch die erfindungsgemäße Zwischenwirbelprothese mit gegeneinander verkippten Prothesenplatten in einer weiteren Position,
- Figur 8b: einen Axialschnitt entlang der Hauptachsen der beiden Prothesenplatten durch die erfindungsgemäße Zwischenwirbelprothese aus Figur 8a mit gegeneinander verkippten Prothesenplatten in einer weiteren Position,
- Figur 9: vergrößerte Darstellung des Axialschnitts gemäß Figur 8a und
- Figur 10: eine schematische Draufsicht auf einen Wirbelkörper mit aufgesetzter Prothesenplatte.

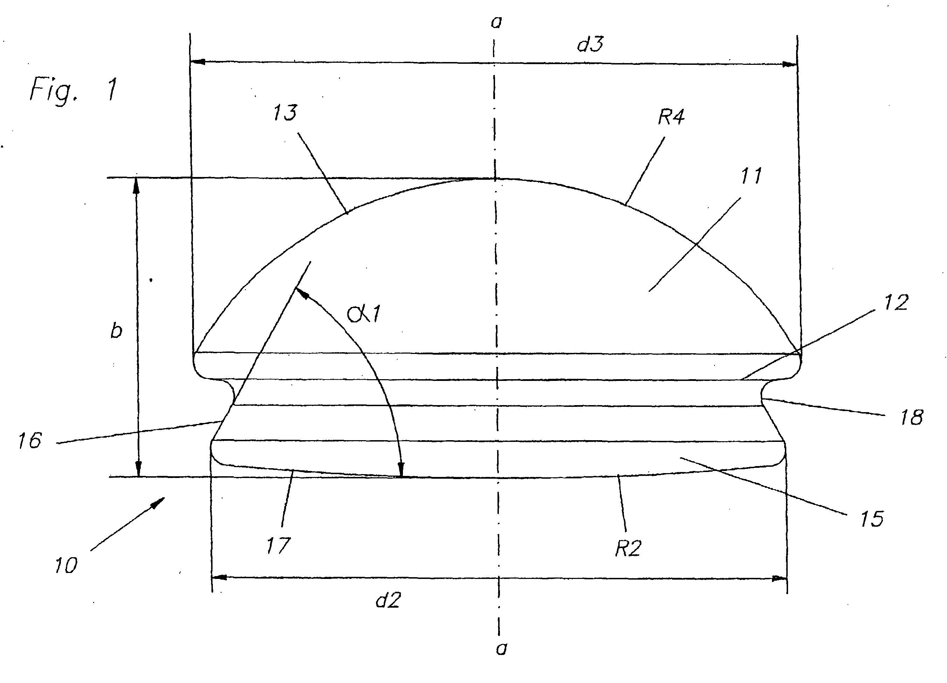

Figur 1 zeigt eine Seitenansicht eines Prothesenkerns 10 gemäß der Erfindung. Der Prothesenkern 10 weist ein Kugelsegment 11 mit einer gewölbten Oberfläche 13 und einer ebenen Oberfläche 12 auf. Das Kugelsegment 11 ist durch einen Radius R4 charakterisiert. Die ebene Oberfläche 12 des Kugelsegments 11 weist einen Durchmesser d3 auf, welcher die größte radiale Ausdehnung des Prothesenkerns 10 angibt.

An der ebenen Oberfläche 12 des Kugelsegments 11 ist ein im Wesentlichen flacher Abschnitt 15 angeordnet. Der flache Abschnitt 15 weist einen im Wesentlichen kreisförmigen Querschnitt mit einem Durchmesser d2 auf. Dadurch ergibt sich ein rotationssymmetrischer Prothesenkern 10. Die Rotationsachse des Prothesenkerns 10 ist dabei in Figur 1 mit a bezeichnet.

Der flache Abschnitt 15 kann auch einen rechteckigen oder quadratischen Querschnitt aufweisen, wobei mit einer derartigen Ausgestaltung des Prothesenkerns 10 weniger gute Simulationsergebnisse der Zwischenwirbelprothese erzielt werden, da sich ein derartiger Prothesenkern 10 nicht frei drehen kann.

Der im Wesentlichen flache Abschnitt 15 weist ausgehend von der ebenen Oberfläche 12 des Kugelsegments 11 eine Außenfläche 16 und in etwa parallel zur ebenen Oberfläche 12 verlaufende Grundfläche 17 auf. Die Außenfläche 16 verläuft gegenüber der Grundfläche 17 in einem Winkel α1, welcher kleiner als 90° ist. Dadurch ergibt sich ein im Wesentlicher flacher Abschnitt 15, welcher sich ausgehend von der ebenen Oberfläche 12 des Kugelsegments 11 konusförmig nach außen bzw. in Richtung der Grundfläche 17 aufweitet.

Die Grundfläche 17 des flachen Abschnitts 15 ist geringfügig konvex gewölbt. Die Wölbung der Grundfläche 17 wird durch einen Radius R2 charakterisiert. Der das Kugelsegment 11 charakterisierende Radius R4 ist dabei wesentlich kleiner, insbesondere fünf bis zehn mal kleiner als der die Wölbung der Grundfläche 17 des flachen Abschnitts 15 charakterisierende Radius R2.

Im vorliegenden Fall beträgt der Radius R4 des Kugelsegments 11 etwa 11 mm. Der die Wölbung der Grundfläche 17 charakterisierende Radius R2 beträgt 100 mm. Der Durchmesser d3 der ebenen Oberfläche 12 des Kugelsegments 11 beträgt etwa 19 mm und stellt die größte radiale Ausdehnung des Prothesenkerns 10 dar. Der Durchmesser d2 des flachen Abschnitts 15 ist grundsätzlich kleiner als der Durchmesser d3 der ebenen Oberfläche 12 und beträgt im vorliegenden Fall etwa 18 mm. Der Prothesenkern 10 weist eine Höhe b auf, welche vorliegend etwa 9 mm beträgt.

Um für jeden Zwischenwirbelraum die geeignete Zwischenwirbelprothese zu verwenden, können selbstverständlich größere oder kleinere Prothesenkerne 10 verwendet werden, bei welchen die vorliegenden Maße entsprechend proportional angepasst werden. Der Durchmesser d2 des im Wesentlichen flachen Abschnitts 15 liegt dabei in der Regel in einem Bereich zwischen 14 mm und 22 mm. Der Radius R4 des Kugelsegments 11 liegt zwischen 8 mm und 14 mm, der Radius R2 der Wölbung der Grundfläche 17 zwischen 80 mm und 120 mm. Die anderen Maße ergeben sich entsprechend.

Der Winkel α1 zwischen der Außenfläche 16 und der Grundfläche 17 des flachen Abschnitts 15 ist grundsätzlich kleiner als 90° und liegt bevorzugt zwischen 50° und 80°. Im vorliegenden Fall beträgt der Winkel α1 etwa 70°. Durch eine derartige schräg gestellte Außenfläche 16 des flachen Abschnitts 15 wird im Übergangsbereich zwischen dem Kugelsegment 11 und dem flachen Abschnitt 15 eine umlaufende Nut 18 gebildet, in welche ein entsprechend geformter Vorsprung einer Prothesenplatte eingreifen kann, um den Prothesenkern 10 zuverlässig in einer Prothesenplatte zu halten.

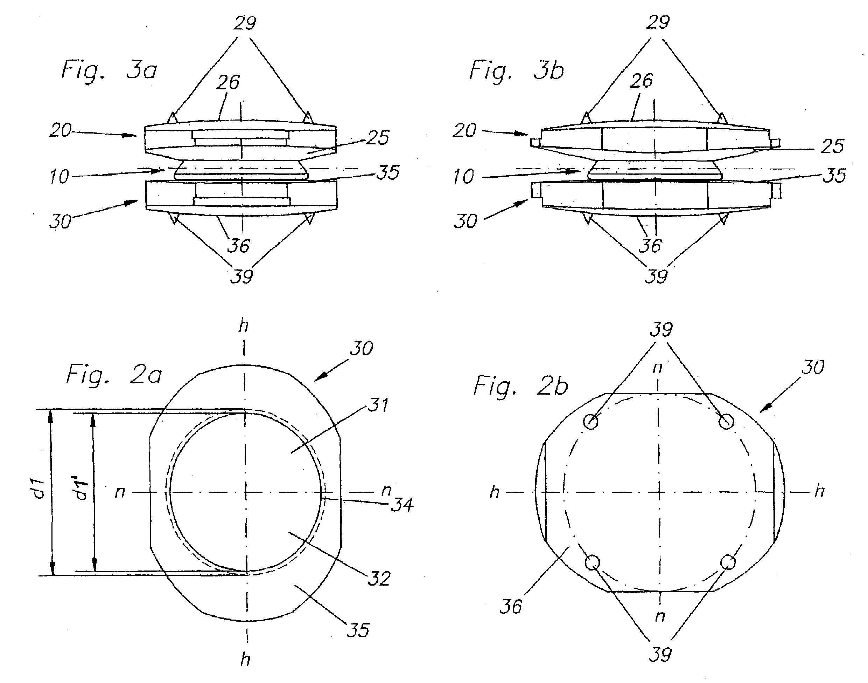

Die Figuren 2a und 2b zeigen eine Draufsicht von Innen und von Außen auf eine zweite Prothesenplatte 30, welche aus einer im Wesentlichen ellipsenförmigen Scheibe mit einer Hauptachse h und einer Nebenachse n besteht. Die Nebenscheitel der ellipsenförmigen Scheibe sind dabei abgeflacht. Die zweite Prothesenplatte 30 weist eine Innenfläche 35 und eine Außenfläche 36 auf. In die Innenfläche 35 ist eine im Wesentlichen ebene Vertiefung 31 eingelassen, welche einen im Wesentlichen runden Querschnitt mit einem Durchmesser d1 aufweist.

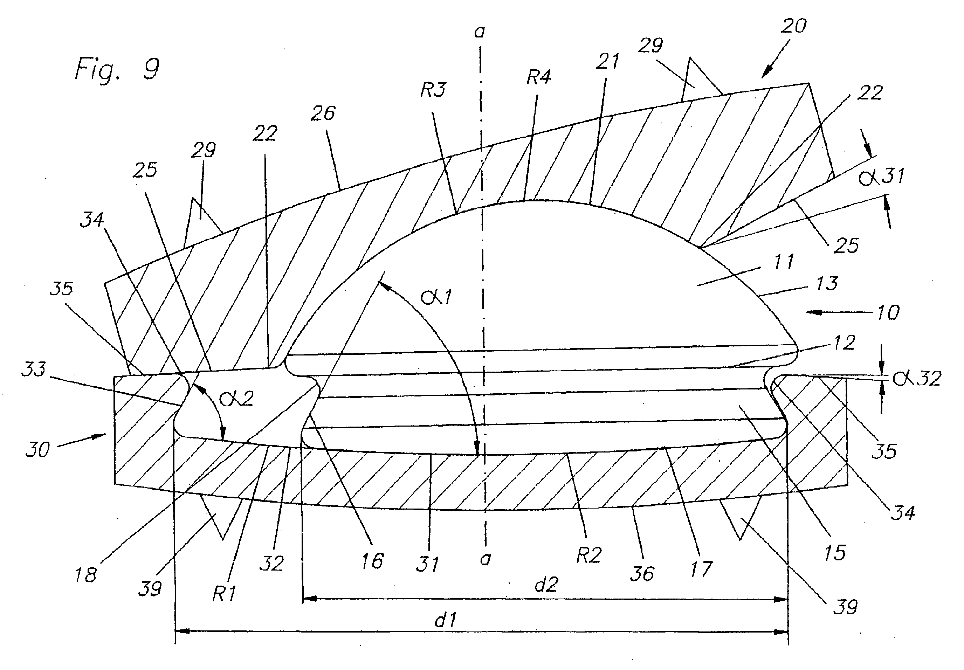

Die im Wesentlichen ebene Vertiefung 31 wird durch eine Grundfläche 32 und eine Außenwand 33 begrenzt (vgl. auch Figur 9). Die Berührungslinie der Innenfläche 35 der zweiten Prothesenplatte 30 mit der Außenwand 33 der ebenen Vertiefung 31 bildet einen Außenrand 34. Die Außenwand 33 bildet mit der Grundfläche 32 der im Wesentlichen ebenen Vertiefung 31 einen Winkel α2 (s. Figur 9), welcher kleiner als 90° ist. Dadurch ist der Durchmesser d1 der Grundfläche 32 der ebenen Vertiefung 31 größer als der Durchmesser d1' des Außenrands 34, so dass sich die im Wesentlichen ebene Vertiefung 31 ausgehend von der Grundfläche 32 konusförmig nach innen verjüngt. Der Durchmesser d1 der ebenen Vertiefung 31 beträgt etwa 23 mm und kann bei entsprechend größeren oder kleineren Zwischenwirbelprothesen zwischen 19 mm und 27 mm liegen.

Die Grundfläche 32 der im Wesentlichen ebenen Vertiefung 31 weist eine konkave Wölbung auf, welche durch einen Radius R1 charakterisiert ist (vgl. auch Figur 9). Der Radius R1 der Wölbung der Grundfläche 32 der im Wesentlichen ebenen Vertiefung 31 liegt zwischen 80 mm und 120 mm und beträgt im vorliegenden Fall etwa 100 mm.

Auf der Außenfläche 36 der zweiten Prothesenplatte 30 sind mehrere, vorliegend vier Zähne 39 angeordnet, über welche die zweite Prothesenplatte 30 in einem Wirbelkörper fixiert wird.

Die Ausdehnung der zweiten Prothesenplatte 30 entlang der Hautachse h beträgt vorliegend 35 mm, die Ausdehnung entlang der Nebenachse n beträgt 27 mm. Auch für die zweite Prothesenplatte 30 gilt, dass für entsprechend größere oder kleinere Zwischenwirbelprothesen die Größe der Prothesenplatte 30 entsprechend, vorzugsweise proportional, anzupassen ist.

Die Figuren 3a und 3b zeigen zwei Seitenansichten, jeweils mit Blickrichtung entlang der Hauptachse h bzw. der Nebenachse n einer Zwischenwirbelprothese bestehend aus einer ersten Prothesenplatte 20, dem bereits beschriebenen Prothesenkern 10 und der bereits beschriebenen zweiten Prothesenplatte 30. Dazu zeigt Figur 9 einen Axialschnitt durch eine Zwischenwirbelprothese im zusammengesetzten Zustand bestehend aus der Prothesenplatte 20, dem Prothesenkern 10 und der Prothesenplatte 30.

Die erste Prothesenplatte 20 weist eine Innenfläche 25 und eine Außenfläche 26 auf. Auf der Innenfläche 25 der ersten Prothesenplatte 20 ist eine kugelschalenförmige Vertiefung 21 eingelassen, welche durch einen Radius R3 charakterisiert ist. Der Radius R3 beträgt vorliegend 11 mm. Die Berührungslinie zwischen der kugelschalenförmigen Vertiefung 21 und der Innenfläche 25 der ersten Prothesenplatte 20 bildet einen Außenrand 22. Auf der Außenfläche 26 der ersten Prothesenplatte 20 sind mehrer Zähne 29 angeordnet, über welche die erste Prothesenplatte 20 in einem Wirbelkörper verankert wird. Die äußeren Dimensionen der ersten Prothesenplatte 20 entsprechen im Übrigen denen der zweiten Prothesenplatte 30.

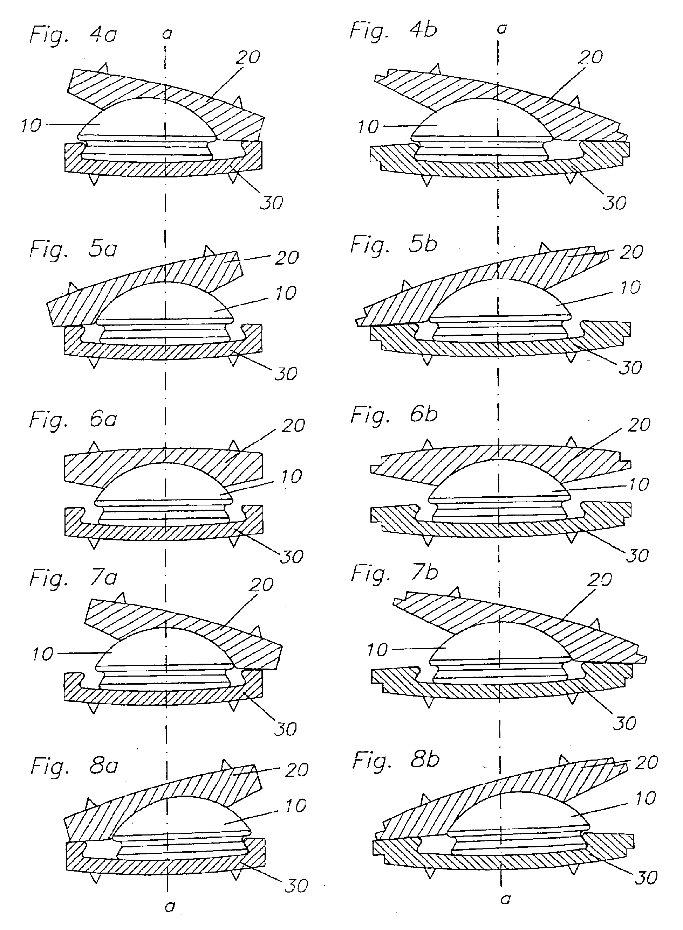

Im zusammengesetzten Zustand der Zwischenwirbelprothese (vgl. Figuren 4 bis 9) greift das Kugelsegment 11 des Prothesenkerns 10 in die kugelschalenförmige Vertiefung 21 der ersten Prothesenplatte 20 ein. Um einen passgenauen Sitz des Prothesenkerns 10 in der ersten Prothesenplatte 20 zu gewährleisten, ist der Radius R4 des Kugelsegments 11 auf den Radius R3 der kugelschalenförmigen Vertiefung 21 abgestimmt.

Der Prothesenkern 10 greift mit dem flachen Abschnitt 15 in die im Wesentlichen ebene Vertiefung 31 der zweiten Prothesenplatte 30 ein. Um eine verschiebliche Bewegung des Prothesenkerns 10 in der zweiten Prothesenplatte 30 zu gewährleisten, ist der Durchmesser d1 der im Wesentlichen ebenen Vertiefung 31 größer als der Durchmesser d2 des flachen Abschnitts 15 ausgebildet (vgl. insbesondere Figur 9).

Zusätzlich ist die Wölbung der Grundfläche 17 des im Wesentlichen flachen Abschnitts 15 auf die Wölbung der Grundfläche 32 der im Wesentlichen ebenen Vertiefung 31 abgestimmt. Dazu ist der Radius R2 des flachen Abschnitts 15 identisch mit dem Radius R1 der Grundfläche 32 der ebenen Vertiefung 31. Eine derartige Abstimmung der Wölbungen gewährleistet, dass der Prothesenkern 10 mit der gesamten Grundfläche 17 auf der Grundfläche 32 der im Wesentlichen ebenen Vertiefung aufliegt.

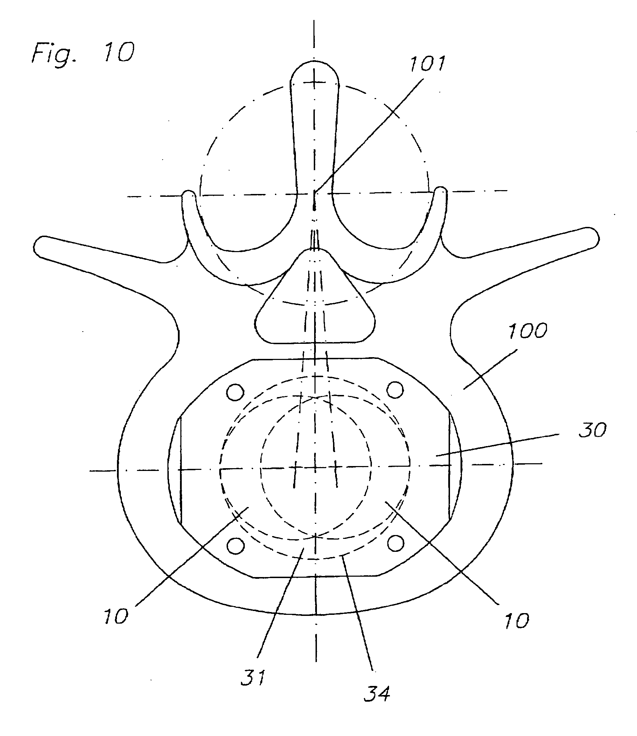

Bei einer Relativbewegung zweier benachbarter Wirbel, an welchen jeweils die erste Prothesenplatte 20 bzw. die zweite Prothesenplatte 30 über die Zähne 29 bzw. die Zähne 39 befestigt sind, wird durch die erfindungsgemäße Zwischenwirbelprothese wie folgt ermöglicht. Die Kippbewegungen der beiden Prothesenplatten 20 und 30 werden durch die Bewegung des Kugelsegments 11 in der kugelschalenförmigen Vertiefung 21 in jeder beliebigen Richtung ermöglicht. Zusätzlich ist der Prothesenkern 10 in der im Wesentlichen ebenen Vertiefung 31 verschiebbar bewegbar. Figur 10 zeigt eine schematische Aufsicht auf einen Wirbelkörper 100, auf welchem die zweite Prothesenplatte 30 befestigt ist. Wie Figur 10 zeigt, wird durch eine Verschiebung des Prothesenkerns 10 in der im Wesentlichen ebenen Vertiefung 31 der Prothesenplatte 30 eine Verschiebung des Drehpunkts 101 der Relativbewegung der Wirbel in den Außenraum außerhalb des Wirbelkörpers 100 ermöglicht. Eine derartige Lage des Drehpunkt 101 kommt der tatsächlichen Lage des Drehpunkts einer Drehbewegung des Oberkörpers somit einer Relativbewegung zwischen zwei benachbarten Wirbeln deutlich näher als die Simulation des Drehpunkts auf der Rotationsachse a des Prothesenkerns 10 bei herkömmlichen Zwischenwirbelprothesen. In der Figur 10 sind dazu zwei verschieden Positionen des Prothesenkerns 10 in der im Wesentlichen ebenen Vertiefung 31 der zweiten Prothesenplatte 30 schematisch angedeutet.

Unterschiedliche relative Positionen der ersten Prothesenplatte 20, der zweiten Prothesenplatte 30 und des Prothesenkerns 10 zeigen die Figuren 4 bis 8. Dabei zeigen die Figuren 4a, 5a, 6a, 7a und 8a jeweils einen Querschnitt entlang der Nebenachse n durch die Zwischenwirbelprothese, während die Figuren 4b, 5b, 6b, 7b und 8b einen Querschnitt entlang der Hauptachse h durch die Zwischenwirbelprothese zeigen. Aus Gründen der Übersichtlichkeit sind in den Figuren 4 bis 8 nur die ersten Prothesenplatte 20, die zweite Prothesenplatte 30 und der Prothesenkern 10 mit Bezugsziffern gekennzeichnet. Die weiteren Bezugszeichen sind der Figur 9, welche eine vergrößerte Darstellung der Figur 8a darstellt, zu entnehmen und analog in sämtliche Figuren 4 bis 8 zu übertragen.

Die Figuren 6a und 6b zeigen die Zwischenwirbelprothese mit zueinander parallel ausgerichteten Prothesenplatten 20 und 30 und dem zwischen der ersten Prothesenplatte 20 und der zweiten Prothesenplatte 30 mittig ausgerichtetem Prothesenkern 10. Wird beispielsweise eine Belastung auf die in den Figuren 6a und 6b rechts von der Rotationsachse a liegenden Enden der ersten Prothesenplatte 20 und zweiten Prothesenplatte 30 ausgeübt, kippt die erste Prothesenplatte 20, wobei das Kugelsegment 11 in der kugelförmigen Vertiefung 12 der ersten Prothesenplatte 20 gleitet. Eine maximale Verkippung ist beispielsweise in den Figuren 7a und 7b dargestellt. Der Prothesenkern 10 verbleibt dabei zunächst in der mittleren Position. Bei weiterer Belastung wird der Prothesenkern 10 ausgehend von der Rotationsachse a nach links gedrückt, wie in den Figuren 4a und 4b gezeigt. Die entsprechenden Relativpositionen bei einer Belastung der ersten Prothesenplatte 20 und der zweiten Prothesenplatte 30 auf den von der Rotationsachse a ausgesehenen linken Seiten der Prothesenplatten ist in den Figuren 5a und 5b bzw. 8a und 8b dargestellt.

Wie besonders deutlich Figur 9 zeigt, greift bei Verschiebung des Prothesenkerns 10 innerhalb der im Wesentlichen ebenen Vertiefung 31 an die Außenwand 33 der Außenrand 34 in die umlaufende Nut 18 des Prothesenkerns 10 ein. Durch die im Winkel α2 schräg zur Grundfläche 32 verlaufenden Außenwand 33 und die im Winkel α1 zur Grundfläche 17 des flachen Abschnitts 15 verlaufenden Außenfläche 16 des flachen Abschnitts 15 wird eine Führung des Prothesenkerns 10 in der ebenen Vertiefung 31 nach Art einer Schwalbenschwanzführung ermöglicht. Dazu sind insbesondere die Winkel al und α2 im Wesentlichen identisch. Dadurch wird der Prothesenkern 10 zuverlässig in der im Wesentlichen ebenen Vertiefung 31 gehalten und kann auch bei starker Belastung nicht aus dem Zwischenraum zwischen der ersten Prothesenplatte 20 und der zweiten Prothesenplatte 30 herausgedrückt werden.

Wie insbesondere in Figur 9 zu sehen ist, verläuft die Innenfläche 25 der ersten Prothesenplatte 20 ausgehend von dem Außenrand 22 der kugelschalenförmigen Vertiefung 21 und die Innenfläche 35 der zweiten Prothesenplatte 30 ausgehend von dem Außenrand 34 der im Wesentlichen ebenen Vertiefung 31 mit einer Neigung α31 bzw. α32 nach außen abfallend. Dadurch wird gewährleistet, dass in der Extremsituation, in welcher die erste Prothesenplatte 20 auf der zweiten Prothesenplatte 30 aufliegt (vgl. z. B. Figur 5a, 5b, 7a und 7b) der Kontakt zwischen der ersten Prothesenplatte 20 und der zweiten Prothesenplatte 30 nicht nur in einem einzigen Punkt, sondern entlang einer radial verlaufenden Linie hergestellt wird. Die Belastung ist somit entlang dieser Auflagelinie verteilt, wodurch zu hohe punktuelle Drücke vermieden und damit einhergehende Beschädigungen der Innenflächen 25, 35 der Prothesenplatten 20, 30 vermieden werden.

Die Neigungen α31 und α32 der Innenflächen 25, 35 der ersten und zweiten Prothesenplatte 20, 30 liegen zwischen 5 und 15°, je nachdem, ein wie großer Kippwinkel zwischen den Prothesenplatten 20, 30 ermöglicht werden soll.

Als Materialien für den Prothesenkern 10 und die beiden Prothesenplatten 20, 30 werden biokompatible Materialien verwendet, da die Prothese in den menschlichen Körper eingesetzt wird. Für die Prothesenplatten 20, 30 werden bevorzugt metallische Werkstoffe, beispielsweise Titan, Titanlegierungen oder Implantatstahl, verwendet. Für den Prothesenkern 10 werden bevorzugt entsprechende Kunststoffe verwendet, da ein Kunststoff weniger hart ist als ein Metall und die federnde Wirkung einer Bandscheibe besser simuliert.

### Bezugszeichenliste

- 10: Prothesenkern
- 11: Kugelsegment
- 12: ebene Oberfläche
- 13: gewölbte Oberfläche
- 15: flacher Abschnitt
- 16: Außenfläche
- 17: Grundfläche (des flachen Abschnitts)
- 18: Nut

- 20: erste Prothesenplatte
- 21: kugelschalenförmige Vertiefung
- 22: Außenrand
- 25: Innenfläche
- 26: Außenfläche
- 29: Zahn

- 30: zweite Prothesenplatte
- 31: ebene Vertiefung
- 32: Grundfläche (der ebenen Vertiefung)
- 33: Außenwand
- 34: Außenrand
- 35: Innenfläche
- 36: Außenfläche
- 39: Zahn

- 100: Wirbelkörper
- 101: Drehpunkt

- R1: Radius (der ebenen Vertiefung)
- R2: Radius (des flachen Abschnitts)
- R3: Radius (der kugelschalenförmigen Vertiefung)
- R4: Radius (des Kugelsegments)

- h: Hauptachse
- n: Nebenachse
- a: Rotationsachse

- d1: Durchmesser (der ebenen Vertiefung)
- d1': Durchmesser (der ebenen Vertiefung am Außenrand)
- d2: Durchmesser (des flachen Abschnitts)
- d3: Durchmesser (des Prothesenkerns)

- α1: Winkel
- α2: Winkel
- α31: Neigung
- α32: Neigung

- b: Höhe

## Patentansprüche

1. Zwischenwirbelprothese bestehend aus einer ersten Prothesenplatte (20) und einer zweiten Prothesenplatte (30) sowie einem zwischen der ersten Prothesenplatte (20) und der zweiten Prothesenplatte (30) angeordneten Prothesenkern (10), wobei die erste Prothesenplatte (20) eine kugelschalenförmige Vertiefung (21) mit einem Radius (R3) aufweist, in welche ein Kugelsegment (11) des Prothesenkerns (10) eingreift, wobei das Kugelsegment (11) des Prothesenkerns eine gewölbte Oberfläche (13) und eine ebene Oberfläche (12) aufweist, wobei die zweite Prothesenplatte (30) eine im Wesentlichen ebene Vertiefung (31) aufweist, in welche ein an der ebenen Oberfläche (12) des Kugelsegments (11) angeordneter im Wesentlichen flacher Abschnitt (15) des Prothesenkerns (11) eingreift, wobei die im Wesentlichen ebene Vertiefung (31) einen im Wesentlichen runden Querschnitt mit einem Durchmesser (d1) aufweist, wobei der im Wesentlichen flache Abschnitt (15) einen im Wesentlichen runden Querschnitt mit einem Durchmesser (d2) aufweist und wobei der Durchmesser (d2) des im Wesentlichen flachen Abschnitts (15) kleiner ist als der Durchmesser (d1) der im Wesentlichen ebenen Vertiefung (31), sodass der im Wesentlichen flache Abschnitt (15) des Prothesenkerns (11) in der im Wesentlichen ebenen Vertiefung (31) verschiebbar bewegbar ist,
**dadurch gekennzeichnet, dass** sich die im Wesentlichen ebene Vertiefung (31) ausgehend von der Grundfläche (32) der im Wesentlichen ebenen Vertiefung (31) konusförmig nach innen zur Bildung eines umlaufenden Vorsprungs verjüngt, dass sich der im Wesentlichen flache Abschnitt (15) ausgehend von der ebenen Oberfläche (12) des Kugelsegments (11) konusförmig nach außen aufweitet, und dass im Übergangsbereich zwischen dem Kugelsegment (11) und dem flachen Abschnitt (15) eine umlaufende Nut (18) gebildet ist, in welche der umlaufende Vorsprung eingreift.

2. Zwischenwirbelprothese nach Anspruch 1,
**dadurch gekennzeichnet, dass** der Durchmesser (d2) des im Wesentlichen flachen Abschnitts (15) etwa 14 mm bis 22 mm, bevorzugt etwa 18 mm beträgt.

3. Zwischenwirbelprothese nach einem der Ansprüche 1 bis 2,
**dadurch gekennzeichnet, dass** der Durchmesser (d1) der im Wesentlichen ebenen Vertiefung (31) 19 mm bis 27 mm, bevorzugt etwa 23 mm, beträgt.

4. Zwischenwirbelprothese nach einem der vorhergehenden Ansprüche,
**dadurch gekennzeichnet, dass** die Grundfläche (32) der im Wesentlichen ebenen Vertiefung (31) eine Wölbung mit einem Radius (R1) aufweist.

5. Zwischenwirbelprothese nach Anspruch 4,
**dadurch gekennzeichnet, dass** der Radius (R1) der Grundfläche (32) der im Wesentlichen ebenen Vertiefung (31) fünf bis zehn mal so groß ist, wie der Radius (R3) der kugelschalenförmigen Vertiefung.

6. Zwischenwirbelprothese nach Anspruch 4 oder 5,
**dadurch gekennzeichnet, dass** der Radius (R1) der Grundfläche (32) der im Wesentlichen ebenen Vertiefung (31) etwa 80 mm bis 120 mm, bevorzugt 100 mm beträgt.

7. Zwischenwirbelprothese nach einem der vorhergehenden Ansprüche,
**dadurch gekennzeichnet, dass** der Radius (R3) der kugelschalenförmigen Vertiefung (21) etwa 8 mm bis 14 mm, bevorzugt etwa 11 mm beträgt.

8. Zwischenwirbelprothese nach einem der vorhergehenden Ansprüche,
**dadurch gekennzeichnet, dass** die Grundfläche (17) des im Wesentlichen flachen Ab-schnitts (15) eine Wölbung mit einem Radius (R2) aufweist.

9. Zwischenwirbelprothese nach Anspruch 10,
**dadurch gekennzeichnet, dass** der Radius (R2) der Wölbung der Grundfläche (17) des im Wesentlichen flachen Abschnitts (15) dem Radius (R1) der Wölbung der Grundfläche (32) der im Wesentlichen ebenen Vertiefung (31) entspricht.

10. Zwischenwirbelprothese nach einem der vorhergehenden Ansprüche,
**dadurch gekennzeichnet, dass** der Winkel (α1) der Außenfläche (16) des flachen Abschnitts (15) gegenüber der Grundfläche (17) des flachen Abschnitts (15) kleiner als 90° ist, bevorzugt zwischen 50 und 80° liegt, besonders bevorzugt etwa 70° beträgt.

11. Zwischenwirbelprothese nach einem der vorhergehenden Ansprüche,
**dadurch gekennzeichnet, dass** der Winkel (α2) zwischen der Außenwand (33) der im Wesentlichen ebenen Vertiefung (31) und der Grundfläche (32) der im Wesentlichen ebenen Vertiefung (31) kleiner als 90° ist, bevorzugt zwischen 50 und 80° liegt, besonders bevorzugt etwa 70° beträgt.

12. Zwischenwirbelprothese nach einem der Ansprüche 10 und 11,
**dadurch gekennzeichnet, dass** der Winkel (α1) dem Winkel (α2) entspricht.

13. Zwischenwirbelprothese nach einem der vorhergehenden Ansprüche,
**dadurch gekennzeichnet, dass** die Innenflächen (25, 35) der ersten und der zweiten Prothesenplatte (20, 30) ausgehend von dem Außenrand (22) der kugelschalenförmigen Vertiefung (21) bzw. vom Außenrand (34) der im Wesentlichen ebenen Vertiefung (31) mit einer Neigung (α31, α32) nach außen abfallend ausgebildet sind.

14. Zwischenwirbelprothese nach Anspruch 13,
**dadurch gekennzeichnet, dass** die Neigungen (α31, α32) 5 bis 15° betragen.

## Claims

1. An intervertebral prosthesis comprising a first prosthesis plate (20) and a second prosthesis plate (30) and a prosthesis core (10) arranged between the first prosthesis plate (20) and the second prosthesis plate (30), wherein the first prosthesis plate (20) has a depression (21) in the form of a spherical shell with a radius (R3) into which a spherical segment (11) of the prosthesis core (10) engages, wherein the spherical segment (11) of the prosthesis core has a convex surface (13) and a flat surface (12), wherein the second prosthesis plate (30) has a substantially flat depression (31) into which a substantially flat portion (15) of the prosthesis core (10) arranged on the flat surface (12) of the spherical segment (11) engages, wherein the substantially flat depression (31) has a substantially round cross-section with a diameter (d1), wherein the substantially flat portion (15) has a substantially round cross-section with a diameter (d2), and wherein the diameter (d2) of the substantially flat portion (15) is smaller than the diameter (d1) of the substantially flat depression (31), so that the substantially flat portion (15) of the prosthesis core (11) [*sic*] is capable of being moved in a displaceable manner in the substantially flat depression (31), **characterized in that** starting from the base area (32) of the substantially flat depression (31) the substantially flat depression (31) tapers inwards in the form of a cone in order to form a continuous projection, starting from the flat surface (12) of the spherical segment (11) the substantially flat portion (15) is widened outwards in the form of a cone, and a continuous groove (18), into which the continuous projection engages, is formed in the transition region between the spherical segment (11) and the flat portion (15).

2. An intervertebral prosthesis according to claim 1, **characterized in that** the diameter (d2) of the substantially flat portion (15) amounts to approximately from 14 mm to 22 mm, preferably approximately 18 mm.

3. An intervertebral prosthesis according to one of claims 1 to 2, **characterized in that** the diameter (d1) of the substantially flat depression (31) amounts to approximately from 19 mm to 27 mm, preferably approximately 23 mm.

4. An intervertebral prosthesis according to any one of the preceding claims, **characterized in that** the base area (32) of the substantially flat depression (31) has a convexity with a radius (R1).

5. An intervertebral prosthesis according to claim 4, **characterized in that** the radius (R1) of the base area (32) of the substantially flat depression (31) is from five to ten times as large as the radius (R3) of the depression in the form of a spherical shell.

6. An intervertebral prosthesis according to claim 4 or 5, **characterized in that** the radius (R1) of the base area (32) of the substantially flat depression (31) amounts to approximately from 80 mm to 120 mm, preferably 100 mm.

7. An intervertebral prosthesis according to any one of the preceding claims, **characterized in that** the radius (R3) of the depression (21) in the form of a spherical shell amounts to approximately from 8 mm to 14 mm, preferably approximately 11 mm.

8. An intervertebral prosthesis according to any one of the preceding claims, **characterized in that** the base area (17) of the substantially flat portion (15) has a convexity with a radius (R2).

9. An intervertebral prosthesis according to claim 8, **characterized in that** the radius (R2) of the convexity of the base area (17) of the substantially flat portion (15) corresponds to the radius (R1) of the convexity of the base area (32) of the substantially flat depression (31).

10. An intervertebral prosthesis according to any one of the preceding claims, **characterized in that** the angle (α1) of the outer face (16) of the flat portion (15) with respect to the base area (17) of the flat portion (15) is less than 90°, is preferably between 50 and 80°, and in a particularly preferred manner amounts to approximately 70°.

11. An intervertebral prosthesis according to any one of the preceding claims, **characterized in that** the angle (α2) between the outer wall (33) of the substantially flat depression (31) and the base area (32) of the substantially flat depression (31) is less than 90°, is preferably between 50 and 80°, and in a particularly preferred manner amounts to approximately 70°.

12. An intervertebral prosthesis according to one of claims 10 and 11, **characterized in that** the angle (α1) corresponds to the angle (α2).

13. An intervertebral prosthesis according to any one of the preceding claims, **characterized in that** starting from the outer edge (22) of the depression (21) in the form of a spherical shell and from the outer edge (34) of the substantially flat depression (31) respectively the inner faces (25, 35) of the first and the second prosthesis plates (20, 30) are made sloping outwards with an inclination (α31, α32).

14. An intervertebral prosthesis according to claim 13, **characterized in that** the inclinations (α31, α32) amount to from 5 to 15°.

## Revendications

1. Prothèse intervertébrale constituée d'une première plaque de prothèse (20) et d'une seconde plaque de prothèse (30) ainsi que d'un noyau de prothèse (10) situé entre la première plaque de prothèse (20) et la seconde plaque de prothèse (30), la première plaque de prothèse (20) comportant une cavité (21) en forme de calotte sphérique ayant un rayon (R3), dans laquelle vient en prise un segment sphérique (11) du noyau de prothèse, le segment sphérique (11) du noyau de prothèse comportant une surface incurvée (13) et une surface plane (12), la seconde plaque de prothèse (30) comportant une cavité (31) essentiellement plane dans laquelle vient en prise en segment (15) du noyau de prothèse (11) essentiellement plan disposé dans la surface plane (12) du segment sphérique (11), la cavité (31) essentiellement plane ayant une section essentiellement circulaire de diamètre (d1), le segment (15) essentiellement plan ayant une section essentiellement circulaire de diamètre (d2), et le diamètre (d2) du segment (15) essentiellement plan étant supérieur au diamètre (d1) de la cavité (31) essentiellement plane de sorte que le segment (15) essentiellement plan du noyau de prothèse (11) puisse se déplacer par coulissement dans la cavité (31) essentiellement plane,
**caractérisée en ce que**
la cavité (31) essentiellement plane s'amincit en forme de cône vers l'intérieur à partir de la surface de base (32) de la cavité (31) essentiellement plane, pour former une saillie périphérique, le segment (15) essentiellement plan s'élargit vers l'extérieur en forme de cône à partir de la surface plane (12) du segment sphérique (11), et, dans la zone de transition entre le segment sphérique (11) et le segment plan (15) est formée une rainure périphérique (18) dans laquelle vient en prise la saillie périphérique.

2. Prothèse intervertébrale conforme à la revendication 1,
**caractérisée en ce que**
le diamètre (d2) du tronçon essentiellement plan (15) est d'environ 14 à 22 mm et de préférence d'environ 18 mm.

3. Prothèse intervertébrale conforme à l'une des revendications 1 et 2,
**caractérisée en ce que**
le diamètre (d1) de la cavité (31) essentiellement plane est de 19 mm à 27 mm de préférence d'environ 23 mm.

4. Prothèse intervertébrale conforme à l'une des revendications précédentes,
**caractérisée en ce que**
la surface de base (32) de la cavité (31) essentiellement plane présente une courbure ayant un rayon (R1).

5. Prothèse intervertébrale conforme à la revendication 4,
**caractérisée en ce que**
le rayon (R1) de la surface de base (32) de la cavité (31) essentiellement plane est de cinq à dix fois le rayon (R3) de la cavité en forme de calotte sphérique.

6. Prothèse intervertébrale conforme à la revendication 4 ou 5,
**caractérisée en ce que**
le rayon (R1) de la surface de base (32) de la cavité (31) essentiellement plane est d'environ 80 mm à 120 mm de préférence de 100 mm.

7. Prothèse intervertébrale conforme à l'une des revendications précédentes,
**caractérisée en ce que**
le rayon (R3) de la cavité (21) en forme de calotte sphérique est d'environ 8 mm à 14 mm, de préférence d'environ 11 mm.

8. Prothèse intervertébrale conforme à l'une des revendications précédentes,
**caractérisée en ce que**
la surface de base (17) du segment (15) essentiellement plan présente une courbure de rayon (R2).

9. Prothèse intervertébrale conforme à la revendication 8,
**caractérisée en ce que**
le rayon (R2) de la courbure de la surface de base (17) du segment (15) essentiellement plan correspond au rayon (R1) de la courbure de la surface de base (32) de la cavité (31) essentiellement plane.

10. Prothèse intervertébrale conforme à l'une des revendications précédentes,
**caractérisée en ce que**
l'angle (α1) entre la face externe (16) du segment plan (15) et à la surface de base (17) du segment plan (15) est inférieur à 90°, de préférence compris entre 50 et 80°, et de façon partiellement préférentielle d'environ 70°.

11. Prothèse intervertébrale conforme à l'une des revendications précédentes,
**caractérisée en ce que**
l'angle (α2) entre la paroi externe (33) da la cavité (31) essentiellement plane et la surface de base (32) de la cavité (31) essentiellement plane est inférieur à 90°, de préférence compris entre 50 et 80° et de façon particulièrement préférentielle de l'ordre de 70°.

12. Prothèse intervertébrale conforme à l'une des revendications 10 et 11,
**caractérisée en ce que**
l'angle (α1) correspond à l'angle (α2).

13. Prothèse intervertébrale conforme à l'une des revendications précédentes,
**caractérisée en ce que**
les surfaces internes (25, 35) de la première et de la seconde plaque de prothèse (20, 30) sont inclinées vers l'extérieur d'un angle (α31, α32) à partir du bord extérieur (22) de la cavité (21) en forme de calotte sphérique ou du bord extérieur (34) de la cavité (31) essentiellement plane.

14. Prothèse intervertébrale conforme à la revendication 13,
**caractérisée en ce que**
les angles (α31, α32) sont de 5 à 15°.
